# EUROPEAN PATENT APPLICATION

(11) **EP 4 316 534 A1**
(43) Date of publication of application: **07.02.2024**
(21) Application number: 22779510.1
(22) Date of filing: 04.02.2022
(51) Int. Cl.: A61L 9/20

(54) **ULTRAVIOLET RAY RADIATION APPARATUS**

(30) Priority: 31.03.2021 JP 2021061925
(71) Applicant: Daikin Industries, Ltd., Osaka-shi, Osaka 530-0001 (JP)
(72) Inventor: SAITO, Tomoki, Osaka-shi, Osaka 530-0001 (JP); TANAKA, Toshio, Osaka-shi, Osaka 530-0001 (JP); OKUMOTO, Mamoru, Osaka-shi, Osaka 530-0001 (JP)
(74) Representative: Hoffmann Eitle
(86) International application number: PCT/JP2022/004375
(87) International publication number: WO 2022/209281

(57) **Abstract**

Contamination in a region around a person who can be a contamination source is reduced. An ultraviolet ray radiation apparatus 1 is provided with a light source 10 that emits ultraviolet rays. This light source 10 emits ultraviolet rays in the range of wavelength from 190 nm to 230 nm. In the ultraviolet ray radiation apparatus 1, the light source 10 emits the ultraviolet rays. This forms an ultraviolet ray region 30, which is a region for receiving the ultraviolet rays, between a first region R1 and a second region R2 adjacent to the first region R1.

## Description

### Technical Field

The present disclosure relates to an ultraviolet ray radiation apparatus.

### Background Art

Patent Document 1 discloses an air curtain formation device that forms an air curtain by vertical airflow between persons facing each other.

### Citation List

### Patent Literature

Patent Document 1: Japanese Utility Model Registration No. 3229800

### Summary of Invention

### Technical Problem

If there is a person who is a potential source of contamination, such as a person infected with an infectious disease, there is a risk of spreading contaminants to the region around the person. When the contaminants are spread, other people may have problems such as acquiring infectious diseases.

An object of the present disclosure is to reduce contamination in a region around a person who is a potential source of contamination.

### Solution to Problem

An ultraviolet ray radiation apparatus of the present disclosure includes: a light source emitting ultraviolet rays, wherein an ultraviolet ray region to be irradiated with the ultraviolet rays is formed between a first region where a person who is a potential source of contamination stays and a second region positioned in a same space as the first region, the second region being desired to be prevented from contamination.

According to the ultraviolet ray radiation apparatus, it is possible to reduce contamination in a region around a person who can be a contamination source.

The ultraviolet ray region may extend in a direction intersecting a direction from the first region to the second region. In this case, it is possible to reduce contamination in a region around a person who can be a contamination source compared to the case where the ultraviolet ray region is formed along the direction from the first region to the second region.

In addition, the light source may emit ultraviolet rays in a wavelength range from 190 nm to 230 nm. In this case, the contamination in the region around the person who is a potential contamination source can be reduced, while minimizing the effects of ultraviolet rays on the human skin and eyes.

Moreover, the light source may be installed above the ultraviolet ray region to emit the ultraviolet rays toward the ultraviolet ray region located below, or the light source may be installed below the ultraviolet ray region to emit the ultraviolet rays toward the ultraviolet ray region located above. In this case, the ultraviolet ray region can be formed by installing the light source on the ceiling or the floor surface.

In addition, each of the first region and the second region may be a region in which a person is expected to stay, and the ultraviolet ray region may be formed between the regions in each of which a person is expected to stay. In this case, wasted ultraviolet ray radiation can be suppressed compared to the case where the ultraviolet ray region is formed between regions where no person is expected to stay.

Further, a control unit controlling the light source to prevent a person from being irradiated with the ultraviolet rays from the light source may be included. In this case, the person can be prevented from being irradiated with the ultraviolet rays from the light source.

Moreover, a change unit changing orientation of the light source may further be included, and thereby the ultraviolet ray region may be formed by changing the orientation of the light source and moving an emission destination of the ultraviolet rays sequentially. In this case, a smaller number of light sources can form the ultraviolet ray region compared to the case where plural fixed light sources are used to form the ultraviolet ray region.

In addition, a movement unit moving the light source may further be included, and thereby the ultraviolet ray region may be formed by moving the light source and moving an emission destination of the ultraviolet rays sequentially. In this case, a smaller number of light sources can form the ultraviolet ray region compared to the case where plural fixed light sources are used to form the ultraviolet ray region.

In addition, a rotation unit rotating the light source may further be included, and thereby the ultraviolet ray region may be formed by rotating the light source and moving an emission destination of the ultraviolet rays sequentially. In this case, a smaller number of light sources can form the ultraviolet ray region compared to the case where plural fixed light sources are used to form the ultraviolet ray region.

Moreover, the ultraviolet ray region may be formed in a fan shape by rotation of the light source around a predetermined axis or movement of the light source along a route with curvature. In this case, the ultraviolet ray region extending in the width as proceeding in the traveling direction of the ultraviolet rays can be formed.

Moreover, an emission destination of the ultraviolet rays may move back and forth and repeat radiation of the ultraviolet rays from one end to the other end of the ultraviolet ray region and radiation of the ultraviolet rays from the other end to the one end. In this case, it is possible to increase the number of times of ultraviolet radiation to the ultraviolet ray region compared to the case where the ultraviolet ray emission destination does not move back and forth.

Moreover, the ultraviolet ray region may be formed between the first region and the second region by radiating the ultraviolet rays in such a way that an emission destination of the ultraviolet rays moves around at least one of the first region and the second region. In this case, the ultraviolet ray region surrounding at least one of the first region and the second region can be formed.

In addition, the ultraviolet ray region may be formed between the first region where a person stays and the second region by radiating the ultraviolet rays in such a way that an emission destination of the ultraviolet rays moves around the person. In this case, the ultraviolet ray region surrounding the person can be formed.

Moreover, a detection unit detecting each person staying in the same space may further be included, and thereby the first region may be a region where one person detected by the detection unit stays, and the second region may be a region where the other person detected by the detection unit stays, and the ultraviolet ray region may be formed between the one person and the other person. In this case, the ultraviolet ray region between the persons can be formed more reliably compared to the case where the ultraviolet ray region is formed without detecting the persons.

### Brief Description of Drawings

FIG. 1 is a diagram illustrating an ultraviolet ray radiation apparatus;
FIG. 2 is a diagram illustrating an example of a hardware configuration of a control device;
FIG. 3 is a diagram showing another configuration example of the ultraviolet ray radiation apparatus;
FIG. 4 is a diagram showing another configuration example of the ultraviolet ray radiation apparatus;
FIG. 5 is a diagram showing another configuration example of the ultraviolet ray radiation apparatus;
FIG. 6 is a diagram showing another configuration example of the ultraviolet ray radiation apparatus;
FIG. 7 is a diagram showing another configuration example of the ultraviolet ray radiation apparatus;
FIG. 8 is a diagram showing another configuration example of the ultraviolet ray radiation apparatus;
FIG. 9 is a diagram showing another configuration example of the ultraviolet ray radiation apparatus; and
FIG. 10 is a diagram showing still another configuration example of the ultraviolet ray radiation apparatus.

### Description of Embodiments

Hereinafter, embodiments will be described with reference to attached drawings.

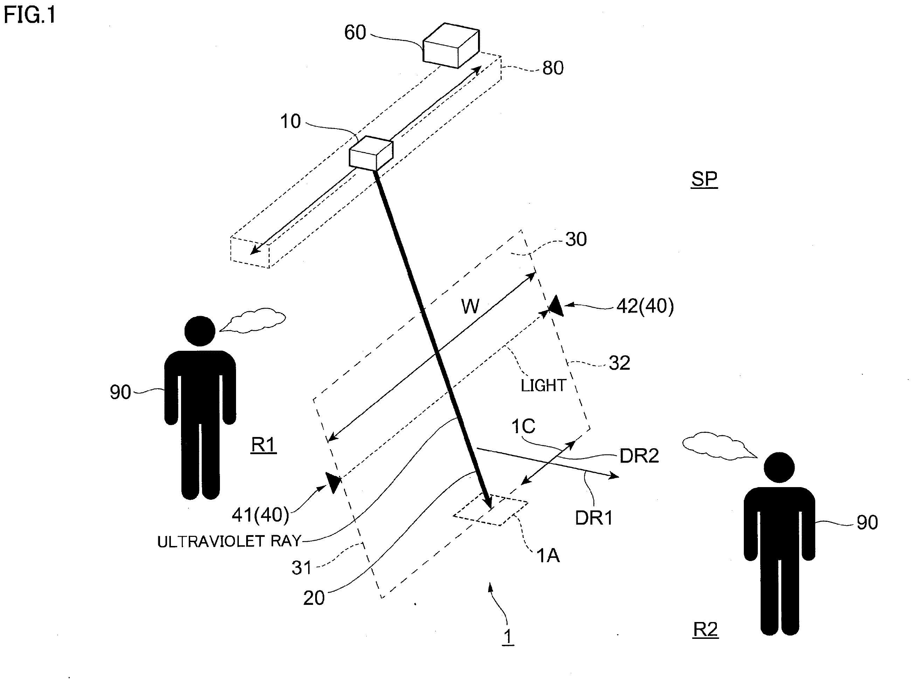

FIG. 1 is a diagram illustrating an ultraviolet ray radiation apparatus 1 related to the present embodiment.

The ultraviolet ray radiation apparatus 1 shown in FIG. 1 includes a light source 10 that emits ultraviolet rays. The light source 10 emits ultraviolet rays in the range of wavelength from 190 nm to 230 nm.

In the case where the ultraviolet rays in the wavelength range from 190 nm to 230 nm are used, it is possible to sterilize viruses and other substances while extremely reducing the effects of ultraviolet rays on human skin and eyes.

The light source 10 is not limited to those emitting ultraviolet rays within the wavelength range from 190 nm to 230 nm, but the light source emitting ultraviolet rays beyond the wavelength range of 190 nm to 230 nm may be used.

In the ultraviolet ray radiation apparatus 1, the light source 10 emits the ultraviolet rays. In the present embodiment, this forms an ultraviolet ray region 30, which is a region to be irradiated with the ultraviolet rays, between a first region R1 and a second region R2 adjacent to the first region R1.

The first region R1 and the second region R2 are located in the same space SP (hereinafter, simply referred to as the "space SP" in some cases).

The sentence "the first region R1 and the second region R2 are located in the same space SP" refers to the situation where the first region R1 and the second region R2 are located in the common space, such as the case where the first region R1 and the second region R2 are located in the same room.

The "space" is not limited to the space located indoors, but also includes the space located outdoors.

The first region R1 is the region where a person 90 who is a potential source of contamination stays. In the present embodiment, the region where the person 90 can stay is the first region R1 where the person 90 who is the potential source of contamination stays. In other words, the region where the person 90 possibly stays is the first region R1.

The person 90 may be infected with infectious diseases, and when the person 90 acquires an infectious disease, the first region R1 is contaminated first. In other words, when the person 90 acquires an infectious disease, the region around the person 90 is contaminated first.

The second region R2 is located in the same space SP as the first region R1, and is adjacent to the first region R1. The second region R2 is the region where prevention of contamination by the source of contamination present in the first region R1 is desired.

The ultraviolet ray region 30 shown in FIG. 1 extends in the direction DR2 intersecting the direction DR1 from the first region R1 to the second region R2.

The intersecting direction DR2 is not limited to the direction orthogonal to the direction DR1 from R1 to R2, but includes any direction other than the orthogonal direction.

In addition, FIG. 1 shows a case where a planar ultraviolet ray region 30 is formed as an example, but the ultraviolet ray region 30 is not limited to the planar shape, and may be formed in a shape of a curved surface.

The ultraviolet ray region 30 may be formed at an angle with respect to the vertical direction as shown in FIG. 1 or along the vertical direction.

The ultraviolet ray region 30 may also be formed along the horizontal direction or at an angle with respect to the horizontal direction.

More specifically, for example, it is also assumed that the first region R1 and the second region R2 are vertically displaced from each other; in this case, the ultraviolet ray region 30 may be formed along the horizontal direction or at an angle with respect to the horizontal direction.

In addition, in the configuration example shown in FIG. 1, the light source 10 is installed above the ultraviolet ray region 30. More specifically, the light source 10 is installed on the ceiling of the space SP. In the present embodiment, ultraviolet rays are emitted downward from the light source 10. This forms the ultraviolet ray region 30 below the light source 10.

However, not limited thereto, the light source 10 may be installed below the location where the ultraviolet ray region 30 is formed, such as the location indicated by the reference sign 1A. In this case, ultraviolet rays are emitted upward, and thereby the ultraviolet ray region 30 is formed above the light source 10.

Furthermore, in the configuration example, a movement mechanism 80 is disposed as an example of a movement unit that moves the light source 10. In the configuration example, the light source 10 is moved by the movement mechanism 80, and thereby an ultraviolet ray emission destination 20 sequentially moves. This forms the ultraviolet ray region 30 that extends in the moving direction of the light source 10 and has the width W in the moving direction.

Specifically, in the present embodiment, the ultraviolet ray emission destination 20 moves back and forth as indicated by the arrow 1C in the figure, to thereby repeat radiation of the ultraviolet rays from one end 31 to the other end 32 of the ultraviolet ray region 30 and radiation of the ultraviolet rays from the other end 32 to the one end 31.

As for the movement mechanism 80, there is no restriction, and any known mechanism may be used.

The movement mechanism 80 is configured with, for example, a pair of pulleys separated from each other, an annular wire running between the pulleys, and a motor as an example of a drive source that rotates one of the pair of pulleys.

When the movement mechanism 80 is used, the light source 10 is attached to the wire. The motor then repeats normal rotation and reverse rotation. This causes the ultraviolet ray emission destination 20 to move back and forth as described above.

In the present embodiment, the light source 10 moves back and forth by the movement mechanism 80. With this, the ultraviolet ray emission destination 20 moves back and forth, and thereby the ultraviolet ray region 30 is irradiated with the ultraviolet rays repeatedly.

The radiation of the ultraviolet rays to the ultraviolet ray region 30 with the width W may be carried out by disposing plural light sources 10 as will be described later; however, as in the present embodiment, when one light source 10 is used, a smaller number of light sources 10 can radiate the ultraviolet rays to the ultraviolet ray region 30.

Here, the "ultraviolet ray region 30" is not limited to the region that is always irradiated with the ultraviolet rays. The "ultraviolet ray region 30" includes regions intermittently irradiated with the ultraviolet rays by sequential movement of the ultraviolet ray emission destination 20.

In the present embodiment, the region where the emission destination 20 passes is also the "ultraviolet ray region 30."

In the configuration example shown in FIG. 1, furthermore, a control device 60 as an example of a control unit that controls the light source 10 is disposed. In the configuration example, a detection sensor 40 as an example of a detection unit that detects that a person 90 enters the ultraviolet ray region 30 is also disposed.

The detection sensor 40 is a so-called transmission-type sensor and includes a detection light source 41 that emits light across the ultraviolet ray region 30 and a light receiving section 42 that receives light emitted from the detection light source 41.

The detection sensor 40 detects that a person 90 enters the ultraviolet ray region 30 when the light receiving section 42 does not receive the light from the detection light source 41.

In the present embodiment, when the detection sensor 40 detects that a person 90 enters the ultraviolet ray region 30, the control device 60 controls the light source 10 so that the person 90 is not irradiated with the ultraviolet rays from the light source 10.

Specifically, the control device 60 turns off the light source 10 or changes the orientation of the light source 10 so that the person 90 in the ultraviolet ray region 30 is not irradiated with the ultraviolet rays from the light source 10.

This prevents the person 90 from being irradiated with the ultraviolet rays from the light source 10.

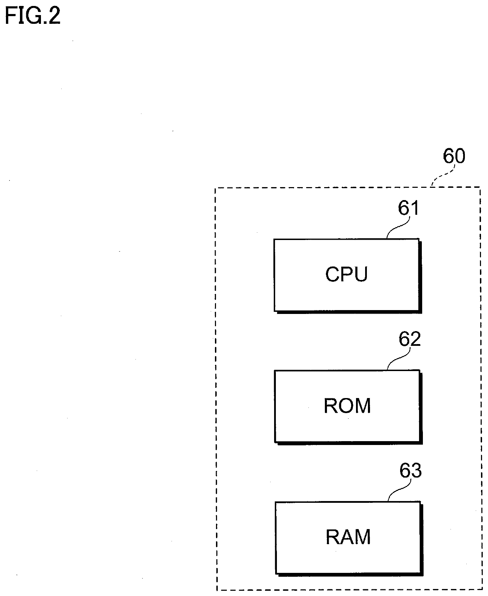

FIG. 2 is a diagram illustrating an example of a hardware configuration of the control device 60.

The control device 60 includes a CPU (Central Processing Unit) 61 as an example of a processor, a ROM (Read Only Memory) 62 storing software for controlling, etc., and a RAM (Random Access Memory) 63 used as a work area.

The CPU 61 can be a multi-core CPU. The ROM 63 may be a rewritable and non-volatile semiconductor memory. The control device 60 is a so-called computer.

Here, the programs to be executed by the CPU 61 can be provided to the control device 60 in the state of being stored in a computer-readable recording medium, such as a magnetic recording medium(a magnetic tape, a magnetic disk, etc.), an optical recording medium (an optical disk, etc.), or a semiconductor memory.

In addition, the programs to be executed by the CPU 61 may be provided to the control device 60 using communication methods such as the Internet.

If there is a person 90 who is a potential source of contamination, such as a person infected with an infectious disease, in the first region R1 shown in FIG. 1, there is a risk of spreading contaminants, such as a virus, to the second region R2, which is a region around the person 90. If the contaminants are spread to the second region R2, a person 90 in the second region R2 may have problems such as acquiring infectious diseases.

In contrast thereto, in the present embodiment, the contaminants are irradiated with ultraviolet rays when passing through the ultraviolet ray region 30. This inactivates the contaminants, and reduces contamination in the second region R2.

The moving speed of the ultraviolet ray emission destination 20 is not limited.

The moving speed of the ultraviolet ray emission destination 20 may be determined in accordance with the amount of contaminants from the first region R1 to the second region R2. The moving speed of the ultraviolet ray emission destination 20 may be set to increase when there are a large amount of contaminants, and decrease when there are a small amount of contaminants.

Each of the first region R1 and the second region R2 is a region where a person 90 is scheduled to stay; in the present embodiment, the ultraviolet ray region 30 is formed between the regions in each of which a person 90 is scheduled to stay.

The "region where a person 90 is scheduled to stay" refers to the region where a person 90 is expected to stop and the person 90 is expected to remain.

Examples of the region where a person 90 is scheduled to stay include a region where chairs or seats are placed. There is no limitation to chairs and seats, and examples of chairs and seats include those placed in mobile bodies such as vehicles, aircraft, and ships. The examples of chairs and seats also include those placed in concert halls, theaters, gymnasiums, and stadiums.

In addition, another example of the region where a person 90 is scheduled to stay includes a region where beds are placed.

In addition, for example, at locations where formation of a procession is expected, each of the locations where a person 90 stands and waits can also be grasped as a region where a person 90 is scheduled to stay, and the ultraviolet ray region 30 may be formed between the region and a region adjacent thereto.

For example, in an escalator, a person 90 stays on each stair and there is each predetermined region where a person 90 stays. In an escalator, movement of contaminants can be suppressed by radiating ultraviolet rays between the stairs to form the ultraviolet ray region 30 between the stairs.

### [Another configuration example]

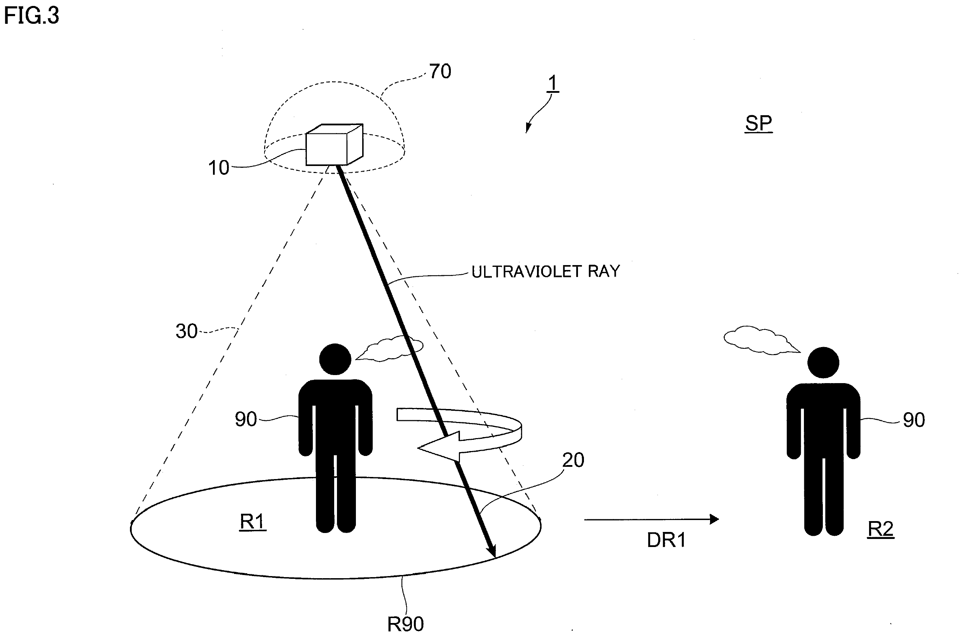

FIG. 3 is a diagram showing another configuration example of the ultraviolet ray radiation apparatus 1.

In the configuration example, a change mechanism 70 is disposed as an example of a change unit that changes the orientation of the light source 10.

In the configuration example, by changing the orientation of the light source 10, the ultraviolet ray emission destination 20 moves sequentially to form the ultraviolet ray region 30. More specifically, in the configuration example, by oscillating the light source 10, the ultraviolet ray emission destination 20 moves sequentially to form the ultraviolet ray region 30.

The movement of the ultraviolet ray emission destination 20 may be carried out by other methods, not by the oscillation of the light source 10. For example, the ultraviolet ray emission destination 20 may be moved by installing a reflective member that reflects the ultraviolet rays from the light source 10 and moving the reflective member or changing the orientation of the reflective member. In other words, without moving the light source 10, the ultraviolet ray emission destination 20 may be moved using members located around the light source 10.

In the configuration example shown in FIG. 3, the ultraviolet ray emission destination 20 moves around the first region R1. Consequently, the circumference of the first region R1 is irradiated with the ultraviolet rays, to thereby form the ultraviolet ray region 30 between the first region R1 and the second region R2.

More specifically, in the configuration example, a cone-shaped ultraviolet ray region 30 surrounding the first region R1 is formed around the first region R1.

In the configuration example, the ultraviolet ray region 30 is formed all around the first region R1, and the spread of contamination is suppressed not only in the direction of DR1 from the first region R1 to the second region R2, but in any direction from the inside of the first region R1 to the outside of the first region R1.

Since the ultraviolet ray region 30 just has to be formed between the first region R1 and the second region R2, the ultraviolet rays may be radiated not only around the first region R1 but also around the second region R2.

In other words, the ultraviolet rays may be radiated so that the ultraviolet ray emission destination 20 moves around the second region R2.

In addition, the ultraviolet rays may be radiated both around the first region R1 and around the second region R2. In other words, the ultraviolet rays may be radiated so that the ultraviolet ray emission destination 20 moves around the first region R1 and around the second region R2.

Moreover, inside the cone-shaped ultraviolet ray region 30 may be or may not be irradiated with the ultraviolet rays.

Similarly, in the configuration example shown in FIG. 1, a location closer to the first region R1 than the ultraviolet ray region 30 may be or may not be irradiated with the ultraviolet rays.

In other words, inside the first region R1, which is separated by the ultraviolet ray region 30 and in which a person 90 stays, may be or may not be irradiated with the ultraviolet rays. When inside the first region R1 is irradiated with the ultraviolet rays, the person 90, who is a potential source of contamination, himself (herself) is irradiated with the ultraviolet rays; thereby sterilizing effects will increase.

In order to ensure safety, when inside the first region R1 is irradiated with the ultraviolet rays, the ultraviolet rays within the wavelength range from 190 nm to 230 nm are radiated.

In the configuration example shown in FIG. 3, the neck of the light source 10 is oscillated, and thereby the ultraviolet ray emission destination 20 moves along the annular route R90.

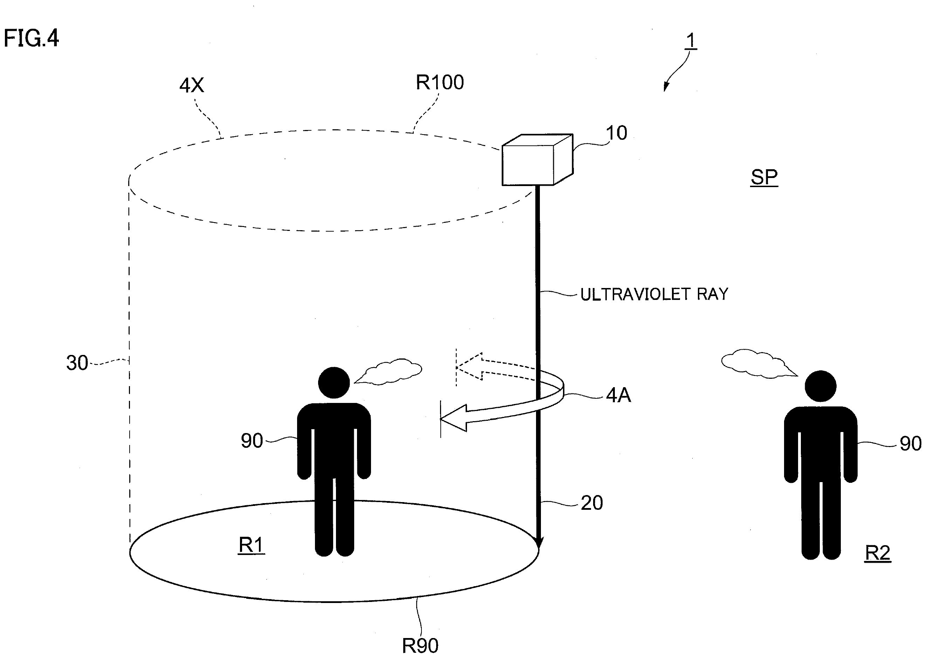

The movement of the emission destination 20 along the annular route R90 is not limited to that caused by oscillation of the light source 10; for example, as shown in FIG. 4 (a diagram showing another configuration example of the ultraviolet ray radiation apparatus 1), the emission destination 20 may be moved by the moving the light source 10 along another annular route R100.

In addition, the route R90 along which the emission destination 20 of the light source 10 moves is not limited to the annular shape, and may be in a shape other than the annular shape, such as an elliptical shape and a rectangular shape.

Moreover, similar to the above, the light source 10 is not limited to being disposed at the upper side of the ultraviolet ray region 30, but may also be disposed on the floor, etc. to form the ultraviolet ray region 30 from below the ultraviolet ray region 30.

In addition, in the above, the case in which the first region R1 and the second region R2 were fixed, and the ultraviolet ray region 30 was also formed at a predetermined location was described as an example.

However, not limited thereto, the first region R1 and the second region R2 may be set in response to positions or movement of persons 90 staying in the space SP.

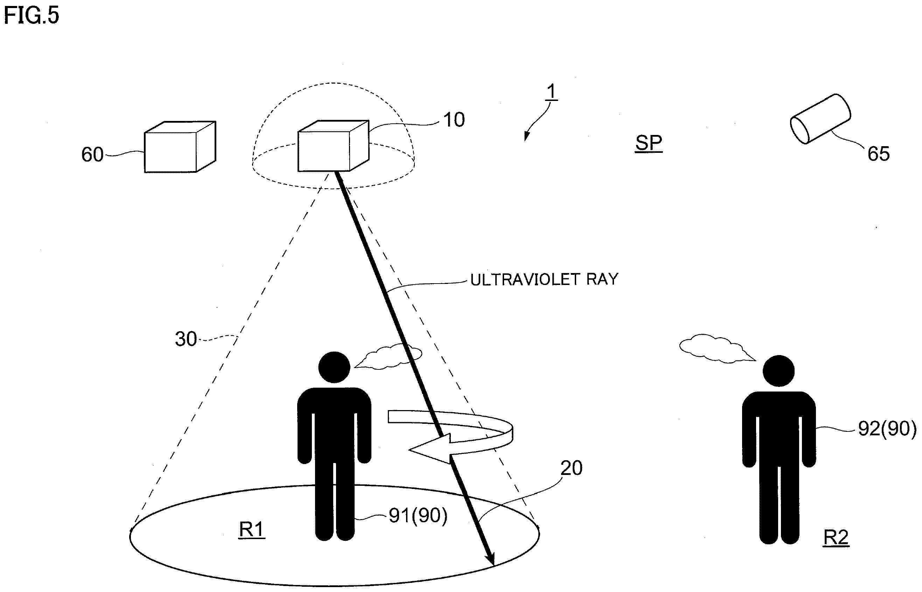

When the first region R1 and the second region R2 are set in accordance with the positions or movement of the persons 90 staying in the space SP, for example, as shown in FIG. 5 (a diagram showing another configuration example of the ultraviolet ray radiation apparatus 1), a camera 65 is installed in the space SP where the persons 90 are staying to capture the state in the space SP.

In this case, the control device 60, which also functions as a detection unit, analyzes the image obtained by the camera 65 and detects each person 90 in the space SP.

The control device 60 then ensures that the ultraviolet ray region 30 is formed between one person 91 who has been detected and the other person 92 who has been detected.

Specifically, the control device 60 controls the orientation of the light source 10 so that the emitted ultraviolet rays are headed between one person 91 who has been detected and the other person 92 who has been detected, to thereby form the ultraviolet ray region 30.

In this case, the first region R1 is a region where one person 91 who has been detected by the control device 60 stays. The second region R2 is a region where the other person 92 who has been detected by the control device 60 stays. In the configuration example, the ultraviolet ray region 30 is formed between one person 91 and the other person 92.

The mode of ultraviolet ray radiation between the one person 91 who has been detected and the other person 92 who has been detected is not limited. For example, as shown in FIG. 5, the ultraviolet rays may be radiated so that the ultraviolet ray emission destination 20 moves around the one person 91, to thereby radiate the ultraviolet rays between the one person 91 and the other person 92.

In addition, for example, the planar-shaped ultraviolet ray region 30 as shown in FIG. 1 may be formed between the one person 91 who has been detected and the other person 92 who has been detected.

Thus, formation of the ultraviolet ray region 30 between the one person 91 who has been detected and the other person 92 who has been detected makes it possible to form the ultraviolet ray region 30 between the persons 90 even in the case where each person 90 moves.

### [Another configuration example]

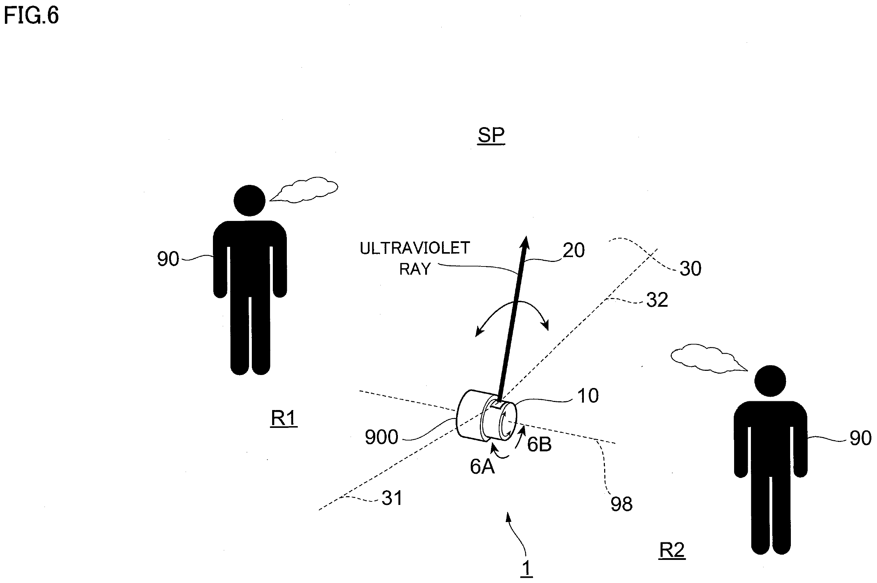

FIG. 6 is a diagram showing another configuration example of the ultraviolet ray radiation apparatus 1.

In the configuration example shown in FIG. 6, the light source 10 rotates around a predetermined axis 98. Furthermore, in the configuration example, a rotation mechanism 900 is disposed as an example of a rotation unit that rotates the light source 10.

In the configuration example, the light source 10 is rotated by the rotation mechanism 900, and thereby the ultraviolet ray emission destination 20 sequentially moves. In addition, in the configuration example, the light source 10 is installed on the floor to emit the ultraviolet rays upwardly.

This forms the ultraviolet ray region 30 in a fan shape above the light source 10, the fan-shaped ultraviolet ray region 30 extending in the width upwardly in the configuration example.

More specifically, in the configuration example, the rotation of the light source 10 in one direction indicated by the reference sign 6A in the figure and the rotation of the light source 10 in the opposite direction indicated by the reference sign 6B in the figure are repeated.

This repeats radiation of the ultraviolet rays from one end 31 to the other end 32 of the fan-shaped ultraviolet ray region 30 and radiation of the ultraviolet rays from the other end 32 to the one end 31.

Note that the light source 10 may be installed above the ultraviolet ray region 30; in this case, the ultraviolet ray region 30 in a fan shape is formed below the light source 10, the fan-shaped ultraviolet ray region 30 extending in the width downwardly.

In the configuration example shown in FIG. 6, the case in which the fan-shaped ultraviolet ray region 30 was formed by the rotation of the light source 10 was described as an example.

The configuration to form the fan-shaped ultraviolet ray region 30 is not limited to the above.

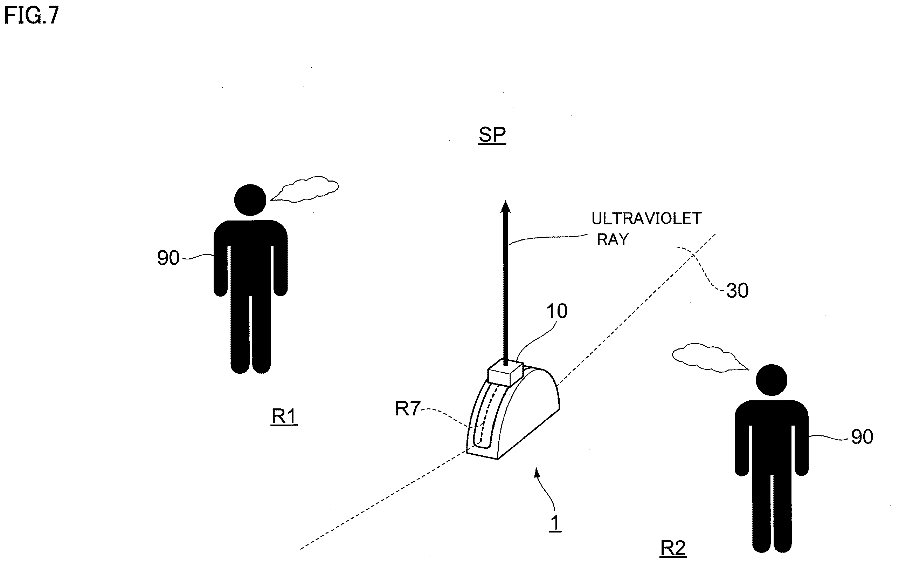

For example, as shown in FIG. 7 (a diagram showing another configuration example of the ultraviolet ray radiation apparatus 1), the fan-shaped ultraviolet ray region 30 may be formed by moving the light source 10 along a route R7 with an upwardly convex curvature.

In addition, though the illustration is omitted, the fan-shaped ultraviolet ray region 30 extending in the width downwardly may be formed by providing a route with a downwardly convex curvature, and moving the light source 10 along the route.

### [Another configuration example]

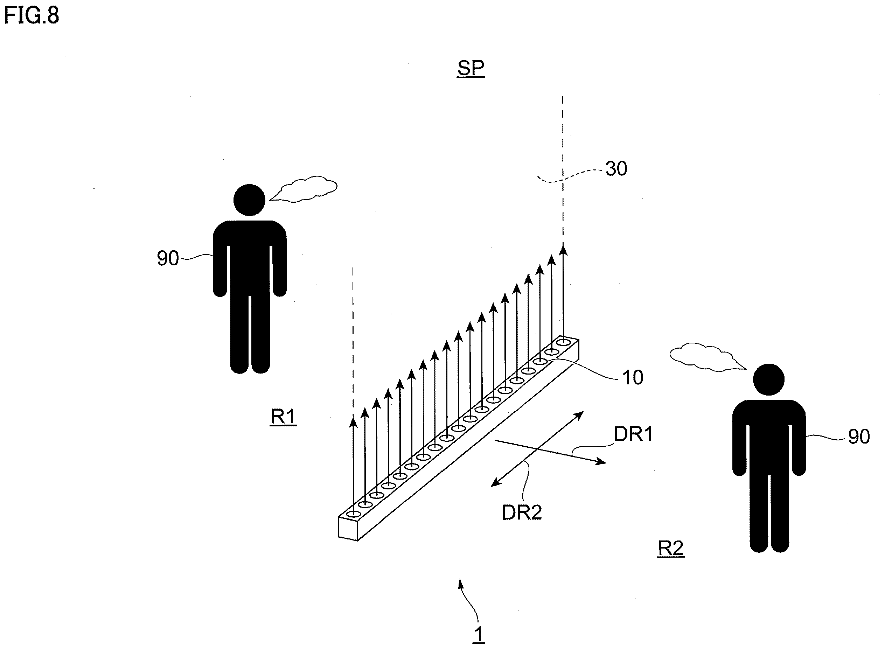

FIG. 8 is a diagram showing another configuration example of the ultraviolet ray radiation apparatus 1.

In the above configuration example, the ultraviolet ray region 30 was formed by moving the ultraviolet ray emission destination 20 sequentially; however, the movement of the emission destination 20 is not essential, and the ultraviolet ray region 30 may be formed by disposing plural light sources 10 as shown in FIG. 8.

In the configuration example, plural light sources 10 are aligned along the direction DR2 intersecting the direction DR1 from the first region R1 to the second region R2. In the configuration example, a planar-shaped ultraviolet ray region 30 can be formed without moving the light source 10.

In the configuration examples shown in FIGS. 1 to 7, the ultraviolet ray region 30 may also be formed by disposing the plural light sources 10.

Specifically, for example, in the configuration example shown in FIG. 1, when the plural light sources 10 are disposed, the plural light sources 10 are arranged in the intersecting direction DR2 shown in FIG. 1, and each light source 10 is disposed so that the light axis thereof is diagonally upward. This forms the ultraviolet ray region 30 at an angle with respect to the vertical direction, which is shown in FIG. 1.

In addition, for example, in the configuration example shown in FIG. 4, when the plural light sources 10 are disposed, the ultraviolet ray region 30 surrounding the first region R1 can be formed by arranging the light sources 10 along the annular line indicated by the reference sign 4X in FIG. 4, for example.

### [Another configuration example]

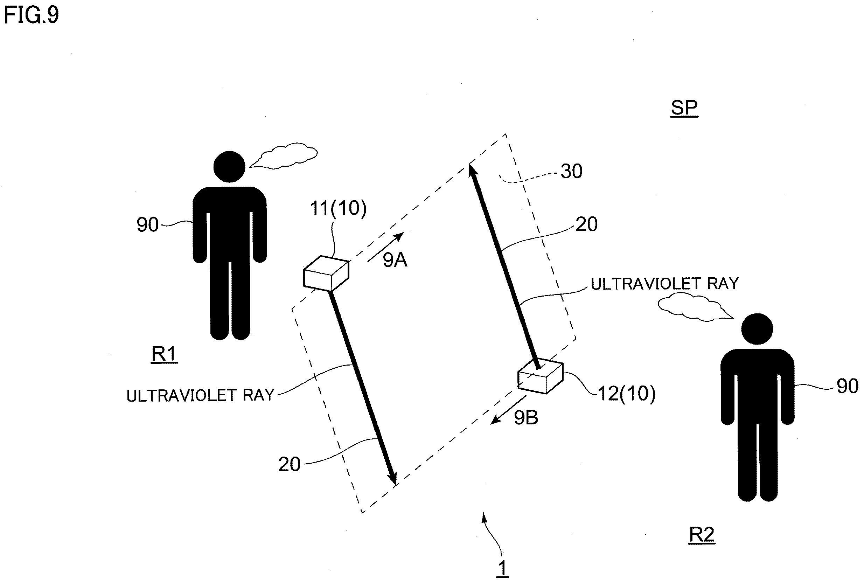

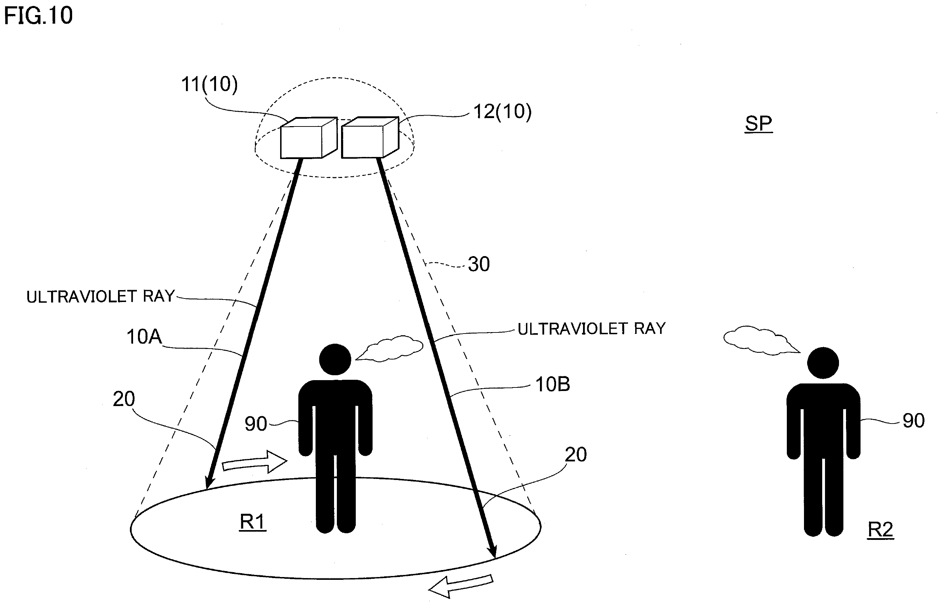

FIGS. 9 and 10 are diagrams each showing another configuration example of the ultraviolet ray radiation apparatus 1.

In the above, the case in which the ultraviolet ray emission destination 20 emitted from the single light source 10 was moved was described as an example; however, the plural light sources 10 may be disposed as shown in FIGS. 9 and 10.

In the configuration example shown in FIG. 9, a first light source 11 and a second light source 12 are disposed as the light source 10. In addition, in the configuration example, when the first light source 11 moves in one direction indicated by the arrow 9A in the figure, the second light source 12 moves in the opposite direction indicated by the arrow 9B in the figure.

In the configuration example shown in FIG. 10, a first light source 11 and a second light source 12 are also disposed.

In the configuration example shown in FIG. 10, the orientation of the direction 10A, in which the ultraviolet rays emitted from the first light source 11 head for, viewed from the first light source 11 and the orientation of the direction 10B, in which the ultraviolet rays emitted from the second light source 12 head for, viewed from the second light source 12 are shifted by 180° °from each other.

In this way, installation of the plural light sources 10 increases the amount of ultraviolet rays radiated to the ultraviolet ray region 30 per unit time, and further reduces the spread of contaminants compared to the case in which the single light source 10 is installed.

Note that, in the configuration examples shown in FIGS. 9 and 10, description was given to the case in which the two light sources 10 were installed; however, there may be three or more light sources 10.

In addition, in the above, the description was given of the case in which the output of the light source 10 was constant as an example; however, the output of the light source 10 may be increased or decreased.

Specifically, for example, when the emission destination 20 passes between the first region R1 and the second region R2 (for example, the location indicated by the reference sign 4A in FIG. 4), the output of the light source 10 is assumed to be the first output. In addition, when the emission destination 20 passes through a location other than between the regions R1 and R2, the output of the light source 10 is reduced and assumed to be the second output smaller than the first output.

In this case, power consumption can be reduced while suppressing the movement of contaminants from the first region R1 to the second region R2.

In addition, for example, the output of the light source 10 may be increased or decreased in response to the degree of contamination in the first region R1.

Specifically, for example, when the number of persons 90 staying in the first area R1 is grasped by analyzing images obtained by the camera 65 (see FIG. 5), and the grasped number of persons is larger than a predetermined threshold, the output of the light source 10 is increased.

In addition, for example, when there is no person 90 at all in the first region R1, the light source 10 is turned off or the output of the light source 10 is decreased.

In addition, for example, the action and state of the person 90 staying in the first region R1 are grasped, and based on the results of the grasp, the output of the light source 10 may be increased or decreased.

Specifically, for example, when a person 90 staying in the first region R1 is not wearing a mask, when the person 90 coughs, or when the person 90 makes a loud voice, the output of the light source 10 is increased.

In other words, when a person 90 staying in the first region R1 takes a specific action or is in a specific state, the output of the light source 10 is increased.

Whether or not the person 90 staying in the first region R1 has taken a specific action or is in a specific state is determined by analyzing images obtained by the camera 65, for example.

In addition, for example, the output of the light source 10 may be increased or decreased upon considering the state of the second region R2. In other words, the output of the light source 10 may be increased or decreased based on the situation of the second region R2.

Specifically, for example, in the case where a person 90 in the second region R2 is more likely to acquire an infectious disease, such as in the case where the person 90 in the second region R2 is an elderly person, the output of the light source 10 is increased. In addition, for example, when there is no person 90 at all in the first region R2, the light source 10 is turned off or the output of the light source 10 is reduced.

Similar to the above, the situation in the second region R2 is grasped, for example, by analyzing the images obtained by the camera 65.

In addition, for example, the moving speed of the ultraviolet ray emission destination 20 may be increased or decreased in response to the degree of contamination in the first region R1. In other words, in response to the degree of contamination in the first region R1, the moving speed, the rotational speed, and the oscillation speed of the light source 10 may be increased or decreased.

Specifically, for example, the number of persons 90 staying in the first area R1 is grasped by analyzing images obtained by the camera 65 (see FIG. 5). When the grasped number of persons is larger than a predetermined threshold, the moving speed of the ultraviolet ray emission destination 20 is increased.

This increases the amount of ultraviolet rays radiated to the ultraviolet ray region 30 per unit time.

In addition, for example, when there is no person 90 at all in the first region R1, the moving speed of the emission destination 20 is decreased, or the emission destination 20 does not move.

In addition, for example, the action and state of the person 90 staying in the first region R1 are grasped, and based on the results of the grasp, the moving speed of the ultraviolet ray emission destination 20 may be increased or decreased. In other words, based results of the grasp, the moving speed, the rotational speed, and the oscillation speed of the light source 10 may be increased or decreased.

Specifically, for example, when a person 90 staying in the first region R1 is not wearing a mask, when the person 90 coughs, or when the person 90 makes a loud voice, the moving speed of the ultraviolet ray emission destination 20 is increased.

In other words, when a person 90 staying in the first region R1 takes a specific action or is in a specific state, the moving speed of the ultraviolet ray emission destination 20 is increased.

Similar to the above, whether or not the person 90 staying in the first region R1 has taken a specific action or is in a specific state is determined by analyzing images obtained by the camera 65, for example.

In addition, for example, the moving speed of the ultraviolet ray emission destination 20 may be increased or decreased upon considering the state of the second region R2. In other words, upon considering the state of the second region R2, the moving speed, the rotational speed, and the oscillation speed of the light source 10 may be increased or decreased.

Specifically, for example, in the case where a person 90 in the second region R2 is more likely to acquire an infectious disease, such as in the case where the person 90 in the second region R2 is an elderly person, the moving speed of the ultraviolet ray emission destination 20 is increased. In addition, for example, when there is no person 90 at all in the second region R2, the moving speed of the ultraviolet ray emission destination 20 is decreased, or the ultraviolet ray emission destination 20 does not move.

Similar to the above, the situation in the second region R2 is grasped, for example, by analyzing the images obtained by the camera 65.

Moreover, in the configuration where the ultraviolet rays are radiated inside the space SP as in the present embodiment, the walls and floors facing the space SP are irradiated with the ultraviolet rays, and thereby the walls and floors are likely to deteriorate.

For this reason, for at least part of the walls and floors, it is preferable to form thereof by materials that have resistance to ultraviolet rays.

Specifically, for example, it is preferable to constitute at least part of the walls and floors constitute by fluorine resin, such as PTFE, PFA, FEP, ETFE, EFEP, CPT, and PCTFE.

In addition, similarly, as for the goods to be placed in the space SP, such as chairs to be placed in the space SP, it is preferable to form at least part thereof by materials that have resistance to ultraviolet rays.

Here, each of the embodiments as described above can be viewed as follows.
(1) An ultraviolet ray radiation apparatus 1 including: a light source 10 emitting ultraviolet rays, wherein an ultraviolet ray region 30 to be irradiated with the ultraviolet rays is formed between a first region R1 where a person 90 who is a potential source of contamination stays and a second region R2 positioned in a same space SP as the first region R1, the second region R2 being desired to be prevented from contamination.
   According to the ultraviolet ray radiation apparatus 1, it is possible to reduce contamination in a region around a person 90 who can be a contamination source.
(2) Here, the ultraviolet ray region 30 may extend in a direction intersecting a direction from the first region R1 to the second region R2. In this case, it is possible to reduce contamination in a region around a person 90 who can be a contamination source compared to the case where the ultraviolet ray region 30 is formed along the direction from the first region R1 to the second region R2.
(3) In addition, the light source 10 may emit ultraviolet rays in a wavelength range from 190 nm to 230 nm. In this case, the contamination in the region around the person 90 who is a potential contamination source can be reduced, while minimizing the effects of ultraviolet rays on the skin and eyes of the person 90.
(4) Moreover, the light source 10 may be installed above the ultraviolet ray region 30 to emit the ultraviolet rays toward the ultraviolet ray region 30 located below, or the light source 10 may be installed below the ultraviolet ray region 30 to emit the ultraviolet rays toward the ultraviolet ray region 30 located above. In this case, the ultraviolet ray region 30 can be formed by installing the light source 10 on the ceiling or the floor surface.
(5) In addition, each of the first region R1 and the second region R2 may be a region in which a person 90 is expected to stay, and the ultraviolet ray region 30 may be formed between the regions in each of which a person 90 is expected to stay. In this case, wasted ultraviolet ray radiation can be suppressed compared to the case where the ultraviolet ray region 30 is formed between regions where no person 90 is expected to stay.
(6) Further, a control device 60 controlling the light source 10 to prevent a person 90 from being irradiated with the ultraviolet rays from the light source 10 may be included. In this case, the person 90 can be prevented from being irradiated with the ultraviolet rays from the light source 10.
(7) Moreover, a change mechanism 70 changing orientation of the light source 10 may further be included, and thereby the ultraviolet ray region 30 may be formed by changing the orientation of the light source 10 and moving an emission destination of the ultraviolet rays sequentially. In this case, a smaller number of light sources 10 can form the ultraviolet ray region 30 compared to the case where plural fixed light sources 10 are used to form the ultraviolet ray region 30.
(8) In addition, a movement mechanism 80 moving the light source 10 may further be included, and thereby the ultraviolet ray region 30 may be formed by moving the light source 10 and moving an emission destination of the ultraviolet rays sequentially. In this case, a smaller number of light sources 10 can form the ultraviolet ray region 30 compared to the case where plural fixed light sources 10 are used to form the ultraviolet ray region 30.
(9) In addition, a rotation mechanism 900 rotating the light source 10 may further be included, and thereby the ultraviolet ray region 30 may be formed by rotating the light source 10 and moving an emission destination of the ultraviolet rays sequentially. In this case, a smaller number of light sources 10 can form the ultraviolet ray region 30 compared to the case where plural fixed light sources 10 are used to form the ultraviolet ray region 30.
(10) Moreover, the ultraviolet ray region 30 may be formed in a fan shape by rotation of the light source 10 around a predetermined axis 98 or movement of the light source 10 along a route R7 with curvature. In this case, the ultraviolet ray region 30 extending in the width as proceeding in the traveling direction of the ultraviolet rays can be formed.
(11) Moreover, an emission destination of the ultraviolet rays may move back and forth and repeat radiation of the ultraviolet rays from one end 31 to the other end 32 of the ultraviolet ray region 30 and radiation of the ultraviolet rays from the other end 32 to the one end 31. In this case, it is possible to increase the number of times of ultraviolet radiation to the ultraviolet ray region 30 compared to the case where the ultraviolet ray emission destination does not move back and forth.
(12) Moreover, the ultraviolet ray region 30 may be formed between the first region R1 and the second region R2 by radiating the ultraviolet rays in such a way that an emission destination of the ultraviolet rays moves around at least one of the first region R1 and the second region R2. In this case, the ultraviolet ray region 30 surrounding at least one of the first region R1 and the second region R2 can be formed.
(13) In addition, the ultraviolet ray region 30 may be formed between the first region R1 where a person 90 stays and the second region R2 by radiating the ultraviolet rays in such a way that an emission destination of the ultraviolet rays moves around the person 90. In this case, the ultraviolet ray region 30 surrounding the person 90 can be formed.
(14) Moreover, a detection unit detecting each person 90 staying in the same space may further be included, and thereby the first region R1 may be a region where one person 90 detected by the detection unit stays, and the second region R2 may be a region where the other person 90 detected by the detection unit stays, and the ultraviolet ray region 30 may be formed between the one person 90 and the other person 90. In this case, the ultraviolet ray region 30 between the persons 90 can be formed more reliably compared to the case where the ultraviolet ray region 30 is formed without detecting the persons 90.

Each of the above-described configurations is not limited to the above-described embodiments and modified examples thereof, and can be changed within the scope not departing from the spirit. In other words, it can be appreciated that various changes in form and detail may be made without departing from the spirit and scope presently or hereafter claimed.

For example, part of each of the above-described configurations may be omitted, or another function may be added to each of the above-described configurations.

In addition, in the above, multiple embodiments are described, but the configurations included in one embodiment may be replaced with those included in another embodiment, or the configurations included in one embodiment may be added to another embodiment.

### Reference Signs List

1 Ultraviolet ray radiation apparatus
10 Light source
30 Ultraviolet ray region
60 Control device
70 Change mechanism
80 Movement mechanism
98 Axis
900 Rotation mechanism
R1 First region
R2 Second region
R7 Route
SP Same space

## Claims

1. An ultraviolet ray radiation apparatus comprising:
a light source emitting ultraviolet rays, wherein
an ultraviolet ray region to be irradiated with the ultraviolet rays is formed between a first region where a person who is a potential source of contamination stays and a second region positioned in a same space as the first region, the second region being desired to be prevented from contamination.

2. The ultraviolet ray radiation apparatus according to claim 1, wherein the ultraviolet ray region extends in a direction intersecting a direction from the first region to the second region.

3. The ultraviolet ray radiation apparatus according to claim 1, wherein the light source emits ultraviolet rays in a wavelength range from 190 nm to 230 nm.

4. The ultraviolet ray radiation apparatus according to claim 1, wherein the light source is installed above the ultraviolet ray region and emits the ultraviolet rays toward the ultraviolet ray region located below, or the light source is installed below the ultraviolet ray region and emits the ultraviolet rays toward the ultraviolet ray region located above.

5. The ultraviolet ray radiation apparatus according to claim 1, wherein
each of the first region and the second region is a region in which a person is expected to stay, and
the ultraviolet ray region is formed between the regions in each of which a person is expected to stay.

6. The ultraviolet ray radiation apparatus according to claim 1, further comprising:
a control unit controlling the light source to prevent a person from being irradiated with the ultraviolet rays from the light source.

7. The ultraviolet ray radiation apparatus according to claim 1, further comprising:
a change unit changing orientation of the light source, wherein
the ultraviolet ray region is formed by changing the orientation of the light source and moving an emission destination of the ultraviolet rays sequentially.

8. The ultraviolet ray radiation apparatus according to claim 1, further comprising:
a movement unit moving the light source, wherein
the ultraviolet ray region is formed by moving the light source and moving an emission destination of the ultraviolet rays sequentially.

9. The ultraviolet ray radiation apparatus according to claim 1, further comprising:
a rotation unit rotating the light source, wherein
the ultraviolet ray region is formed by rotating the light source and moving an emission destination of the ultraviolet rays sequentially.

10. The ultraviolet ray radiation apparatus according to claim 1, wherein the ultraviolet ray region is formed in a fan shape by rotation of the light source around a predetermined axis or movement of the light source along a route with curvature.

11. The ultraviolet ray radiation apparatus according to claim 1, wherein an emission destination of the ultraviolet rays moves back and forth and repeats radiation of the ultraviolet rays from one end to the other end of the ultraviolet ray region and radiation of the ultraviolet rays from the other end to the one end.

12. The ultraviolet ray radiation apparatus according to claim 1, wherein the ultraviolet ray region is formed between the first region and the second region by radiating the ultraviolet rays in such a way that an emission destination of the ultraviolet rays moves around at least one of the first region and the second region.

13. The ultraviolet ray radiation apparatus according to claim 1, wherein the ultraviolet ray region is formed between the first region where the person stays and the second region by radiating the ultraviolet rays in such a way that an emission destination of the ultraviolet rays moves around the person.

14. The ultraviolet ray radiation apparatus according to claim 1, further comprising:
a detection unit detecting each person staying in the same space, wherein
the first region is a region where one person detected by the detection unit stays, and the second region is a region where the other person detected by the detection unit stays, and
the ultraviolet ray region is formed between the one person and the other person.
